# EUROPÄISCHE PATENTANMELDUNG

(11) **EP 2 016 958 A2**
(43) Veröffentlichungstag der Anmeldung: **21.01.2009**
(21) Anmeldenummer: 08159443.4
(22) Anmeldetag: 01.07.2008
(51) Int. Cl.: A61L 31/02, A61L 31/08

(54) **Stent mit einer Beschichtung oder Füllung einer Kavität**

(30) Priorität: 20.07.2007 DE 102007034019
(71) Anmelder: BIOTRONIK VI Patent AG, 6341 Baar (CH)
(72) Erfinder: Wittchow, Eric, 90403, Nürnberg (DE)
(74) Vertreter: Lindner-Vogt, Karin L.

(57) **Zusammenfassung**

Die Erfindung betrifft einen Stent aus einem metallischen Grundkörper und mit einer SiO₂- oder silikathaltigen Beschichtung oder Füllung einer Kavität.

## Beschreibung

Die Erfindung betrifft einen Stent aus einem metallischen Grundkörper und mit einer Beschichtung oder Füllung einer Kavität sowie ein dazugehöriges Herstellungsverfahren.

### Technologischer Hintergrund und Stand der Technik

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die tragende Umfangswandung wird in der Regel von einer gitterartigen Tragstruktur gebildet, die es erlaubt, den Stent in einem komprimierten Zustand mit kleinem Außendurchmesser bis zur behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist.

Der Stent besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Für die Zwecke der vorliegenden Erfindung sind lediglich metallische Implantatwerkstoffe für Stents von Interesse. Biokompatible Metalle und Metalllegierungen für Permanentimplantate umfassen rostfreie Stähle (z. B. 316L), Kobaltbasislegierungen (z. B. L605, CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiAl6V4 oder TiAl6Nb7) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen.

Eine biologische Reaktion auf metallische Elemente hängt ab von der Konzentration, Einwirkdauer und Art der Zuführung. Häufig führt die Gegenwart eines Implantatwerkstoffs zu Entzündungsreaktionen, deren Auslöser mechanische Reize, chemische Stoffe aber auch Stoffwechselprodukte sein können. Der Entzündungsvorgang ist in der Regel begleitet von der Einwanderung neutrophiler Granulozyten und Monozyten durch die Gefäßwände, der Einwanderung von Lymphozyten-Effektorzellen unter Bildung spezifischer Antikörper gegen den Entzündungsreiz, der Aktivierung des Komplementsystems unter Freisetzung von Komplementfaktoren, die als Mediatoren wirken, und letztendlich der Aktivierung der Blutgerinnung gefolgt wird. Eine immunologische Reaktion ist meist eng mit der Entzündungsreaktion verbunden und kann zur Sensibilisierung und Allergiebildung führen. Bekannte metallische Allergene umfassen beispielsweise Nickel, Chrom und Kobalt, die auch in vielen chirurgischen Implantaten als Legierungskomponenten Verwendung finden. Ein wesentliches Problem der Stentimplantation in Blutgefäße ist die In-Stent Restenose aufgrund eines überschießenden neointimalen Wachstums, das durch eine starke Proliferation der arteriellen glatten Muskelzellen und eine chronische Entzündungsreaktion hervorgerufen wird.

Bekannt ist, dass sich ein höheres Maß an Biokompatibilität und damit eine Verbesserung der Restenoserate erreichen lässt, wenn metallische Implantatwerkstoffe mit Beschichtungen aus besonders gewebsverträglichen Materialien versehen werden. Diese Materialien sind zumeist organischer oder synthetisch-polymerer Natur und teils natürlichen Ursprungs. Bisherige Strategien zur Vermeidung der Restenose konzentrieren sich zumeist darauf, die Proliferation durch Medikation, z. B. Behandlung mit Cytostatika, zu hemmen.

Trotz der bestehenden Fortschritte besteht noch immer ein hohes Bedürfnis, eine bessere Integration des Stents in sein biologisches Umfeld zu erreichen und dadurch die Restenoserate zu senken.

### Erfindungsgemäße Lösung

Nach einem ersten Aspekt der Erfindung werden eine oder mehrere der oben geschilderten Probleme durch einen Stent aus einem metallischen Grundkörper und mit einer SiO₂-oder silikathaltigen Beschichtung oder Füllung einer Kavität gelöst.

Der Erfindung liegt die Erkenntnis zugrunde, dass in einem gesunden Organismus ein Gleichgewicht aus Zellvermehrung (Proliferation) und Zellsterben (Apoptose) besteht. Kommt es zu einer Restenose nach Stentimplantation ist das Gleichgewicht beider Prozesse gestört und die Proliferation gewinnt überhand über den natürlichen Zelltod. Die bisherigen Strategien zur Vermeidung der Restenose versuchen die Proliferation zu hemmen. Histologische Präparate stenosierter Gefäße konnten jedoch keine erhöhte Konzentration an Proliferationsmarkern gegenüber dem umliegenden Gewebe belegen. Dies stützt die Annahme, dass die Apoptose weniger effektiv erfolgt als in gesundem Gewebe. Hier setzt die Erfindung an: Das Ungleichgewicht soll durch Steigerung der Apoptoserate ausgeglichen werden. Der Vorteil gegenüber der Proliferationshemmung ist unter anderem die Vermeidung einer Akkumulierung neointimaler Zellen ohne dabei die notwenige Gewebeabdeckung des Stens zu verzögern.

Es hat sich nun überraschenderweise gezeigt, dass der Einsatz von Siliziumdioxid oder Silikaten als Bestandteil einer Beschichtung oder Füllung einer Kavität eines metallischen Stents zur Steigerung der Apoptose führt. Der Einsatz dieser anorganischen Substanzen hat den besonderen Vorteil, dass eine hohe Haftung mit dem metallischen Grundkörper erreicht werden kann und die anorganischen Verbindungen thermisch stabil und wenig reaktiv sind, so dass die Herstellung und Sterilisation des Stents vereinfacht ist. Der den positiven Einfluss von Siliziumdioxid und Silikaten auf die Apoptose zugrunde liegende Wirkmechanismus ist weitgehend noch ungeklärt. Vermutlich wird das direkt den apoptotischen Vorgang auslösende Enzym Caspase-3 über eine Änderung der Mitochondrien-Permeabilität aktiviert.

Siliziumdioxid ist der Sammelbegriff für chemische Verbindungen mit der Summenformel SiO₂. Für die Zwecke der Erfindung ist die kristalline Modifikation des SiO₂ offenbar jedoch nur von untergeordneter Bedeutung; wesentlich ist die die apoptotischen Vorgänge in biologischen Systemen beschleunigende Eigenschaft des Materials.

Der Begriff Silikate steht für eine Verbindung aus Silizium und Sauerstoff mit einem oder mehreren Metallen und eventuell noch Hydroxylionen. Auch hier ist die kristalline Modifikation und die Wahl des Metalls beziehungsweise die Gegenwart von Hydroxylionen ersten Untersuchungen zur Folge von untergeordneter Bedeutung mit Hinsicht auf die gewünschte biologische Wirkung.

Das die Apoptose fördernde Material kann Bestandteil einer Beschichtung sein oder die Beschichtung besteht gänzlich aus dem Material. Die Beschichtung kann unmittelbar auf dem Grundkörper des Stents aufgebracht sein oder es sind weitere Schichten dazwischen vorgesehen. Alternativ kann das die die Apoptose fördernde Material Bestandteil einer Kavitätenfüllung sein. Die Kavität befindet sich in der Regel an der Oberfläche des Stents; bei Stents mit einem biodegradierbaren Grundkörper kann die Kavität auch im Innern des Grundkörpers angeordnet sein, so dass die Freisetzung des Material erst nach Freilegung erfolgt. Vorzugsweise enthält die Beschichtung oder Füllung 0,1 bis 10 µg freies oder gebundenes Silizium pro 1 mm Stentlänge.

Nach einer ersten bevorzugten Ausführungsform der Erfindung enthält die Beschichtung oder Füllung Nanopartikel aus SiO₂ oder Silikat. Die Nanopartikel weisen vorzugsweise eine Partikelgröße im Bereich von 5 - 75 nm auf und können in eine polymere Matrix, insbesondere eine polyurethanhaltige polymere Matrix, eingebettet sein. Der Einsatz von Nanopartikel scheint aufgrund der sehr großen Oberfläche der Partikel, die für eine Wechselwirkung mit dem biologischen System zur Verfügung stehen, für die erfindungsgemäßen Zwecke besonders geeignet.

Eine zweite bevorzugte Ausführungsform der Erfindung sieht vor, dass die Beschichtung ein geschlossener Film aus SiO₂ oder Silikat ist. Der Film aus SiO₂ oder Silikat kann eine Dicke im Bereich von 1 bis 15 µm aufweisen. Nach dieser Ausführungsform wird demnach der metallische Grundkörper des Stents direkt oder gegebenenfalls über weitere Zwischenschichten so aufgetragen, dass er eine Oberfläche des Stents bedeckt. Im Falle korrodierbarer Implantate ist mit einer zumeist gewünschten Verzögerung der Degradation durch die relativ inerte Beschichtung zu rechnen. Während der Implantation des Stents bilden sich in der Beschichtung Mikrorisse, so dass der Degradation des Grundkörpers im expandierten Zustand des Stents nichts mehr im Wege steht.

Nach einer weiteren bevorzugten Ausführungsform, die mit den beiden vorgenannten Ausführungsformen realisierbar ist, weist der metallische Grundkörper eine poröse Oberfläche auf, die mit der SiO₂- oder silikathaltigen Beschichtung bedeckt ist. Mit anderen Worten, die zugänglichen Poren der porösen Oberfläche des metallischen Grundkörpers enthalten die oben genannten Nanopartikel, sind mit einer die Nanopartikel enthaltenen polymeren Matrix beschichtet oder die zugänglichen Poren sind von einem Film aus SiO₂ oder Silikat bedeckt. Hierdurch kann die Kontaktfläche zum biologischen System vergrößert werden und es können die erfindungsgemäß gewünschten Effekte auf die Apoptose verstärkt werden.

Vorzugsweise ist das metallischen Grundgerüst aus Magnesium, einer biokorrodierbaren Magnesiumlegierung, Reineisen, einer biokorrodierbaren Eisenlegierung, einer biokorrodierbaren Wolframlegierung, einer biokorrodierbaren Zinklegierung oder einer biokorrodierbaren Molybdänlegierung. Die genannten biokorrodierbaren metallischen Werkstoffe sind zumindest weitgehend chemisch inert gegenüber SiO₂ und Silikaten, so dass ein negativer Einfluss auf die Degradation des Stents nicht zu erwarten ist.

Als biokorrodierbar im Sinne der Erfindung werden Legierungen und Elemente bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist.

Unter Magnesiumlegierung, Eisenlegierung, Zinklegierung, Molybdänlegierung oder Wolframlegierung wird vorliegend ein metallisches Gefüge verstanden, dessen Hauptkomponente Magnesium, Eisen, Zink, Molybdän oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Die Legierung ist so in ihrer Zusammensetzung zu wählen, dass sie biokorrodierbar ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl2 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/I). Eine Probe der zu untersuchenden Legierung wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Ein zweiter Aspekt der Erfindung betrifft die Bereitstellung eines Verfahrens zur Herstellung einer SiO₂-haltigen Beschichtung auf einem Stent aus einem metallischen Grundkörper. Das Verfahren umfasst die Schritte:
(i) Bereitstellen des Stents aus einem metallischen Grundkörper;
(ii) Kontaktieren einer Oberfläche des Grundkörpers mit einer wässrigen kolloiden Dispersion, die amorphe SiO₂-Partikel mit einer mittleren Teilchengröße im Bereich von 5 - 75 nm enthält; und
(iii) gleichzeitig oder im Anschluss an Schritt (ii), thermische Behandlung des Stents zumindest im Bereich der Kontaktfläche unter Ausbildung der SiO₂-haltigen Beschichtung.

Mit dem genannten Verfahren lassen sich in besonders einfacher Weise SiO₂-haltige Beschichtungen auf Stents aus metallischen Grundkörpern erzeugen.

Vorzugsweise wird eine wässrige kolloide Dispersion verwendet, die nur die amorphen SiO₂-Partikel enthält und der Schritt (iii) wird so durchgeführt, dass sich ein Film aus SiO₂ bildet. Nach dieser Variante kommt es demnach zur Agglomeration der in der Dispersion enthaltenen SiO₂-Partikel an der Oberfläche des Implantats unter Ausbildung eines SiO₂-Films.

Alternativ kann im Schritt (ii) eine wässrige kolloide Dispersion verwendet werden, die Polyurethane und einen Polyisocyanat-Härter enthält und der Schritt (iii) wird so durchgeführt, dass sich eine polymere Matrix aus Polyurethan bildet, in die Nanopartikel aus SiO₂ eingebettet sind. Mit anderen Worten, durch die Gegenwart der Polyurethanmatrix wird die Agglomeration des SiO₂-Partikel auf der Oberfläche des metallischen Grundkörpers verhindert und es bilden sich anstelle dessen Nanopartikel aus SiO₂.

Vorzugsweise weist der metallische Grundkörper des Stents eine poröse Oberfläche auf und Schritt (ii) wird so durchgeführt, dass die Dispersion in die Poren der porösen Oberfläche eindringt. Mit anderen Worten, eine innere Oberfläche der zugänglichen Poren wird von einem SiO₂-Film beziehungsweise einer polymeren Matrix mit SiO₂-Nanopartikeln bedeckt.

Nachfolgend wird die Erfindung anhand eines Ausführungsbeispiels näher erläutert.

Ein Stent aus der biodegradierbaren Magnesiumlegierung WE43 (nach ASTM) wurde entfettet und getrocknet.

Folgende Lösungen/Dispersionen wurden bereitgestellt:
(A) Eine kolloid-disperse Lösung von amorphen Siliziumdioxidpartikeln mit einer durchschnittlichen Partikelgröße von 6 nm und in einer Konzentration von 15% (erhältlich unter dem Handelsnamen Levasil 500/15% bei der Firma H.C. Starck).
(B) Eine ca. 39.5 %ige Polyurethan-Dispersion mit blockierten Isocyanat-Gruppenals Härterkomponente (erhältlich unter dem Handelsnamen Bayhydur 5140 bei der Firma Bayer MaterialScience).

Die Lösungen/Dispersionen (A) und (B) wurden im Verhältnis 1 zu 1 vereinigt. Durch die Blockierung der Isocyanatgruppen findet bei Raumtemperatur noch keine Vernetzung statt. Der Stent wurde mit der vereinigten Lösung besprüht. Danach wurde bei 150 °C für 30 Minuten getrocknet.

Es wurden je drei Stents mit der silikathaltigen Beschichtung pro Versuchstier (Hausschwein) implantiert. Nach 14 Tagen beziehungsweise 28 Tagen wurden die Koronargefäße angiographiert, explantiert und histologisch bewertet. Es zeigte sich, dass die Fläche der Intimaneubildung gegenüber unbeschichteten Stents aus der Magnesiumlegierung WE43 deutlich verringert war.

## Patentansprüche

1. Stent aus einem metallischen Grundkörper und mit einer SiO₂- oder silikathaltigen Beschichtung oder Füllung einer Kavität.

2. Stent nach Anspruch 1, bei dem die Beschichtung oder Füllung Nanopartikel aus SiO₂- oder Silikat enthält.

3. Stent nach Anspruch 2, bei dem die Nanopartikel eine mittlere Teilchengröße im Bereich von 5 bis 75 nm aufweisen.

4. Stent nach Anspruch 2 oder 3, bei dem die Nanopartikel in eine polymere Matrix eingebettet sind.

5. Stent nach Anspruch 4, bei dem die Matrix Polyurethan enthält.

6. Stent nach Anspruch 1, bei dem die Beschichtung ein geschlossener Film aus SiO₂ oder Silikat ist.

7. Stent nach Anspruch 6, bei dem der Film aus SiO₂ oder Silikat eine Dicke im Bereich von 1 bis 15 µm aufweist.

8. Stent nach einem der vorhergehenden Ansprüchen, bei dem der metallische Grundkörper eine poröse Oberfläche aufweist, die mit der SiO₂- oder silikathaltigen Beschichtung bedeckt ist.

9. Stent nach einem der vorhergehenden Ansprüchen, bei dem das metallische Grundgerüst des Stents aus Magnesium, einer biokorrodierbaren Magnesiumlegierung, Reineisen, einer biokorrodierbaren Eisenlegierung, einer biokorrodierbaren Wolframlegierung, einer biokorrodierbaren Zinklegierung oder einer biokorrodierbaren Molybdänlegierung besteht.

10. Verfahren zur Herstellung einer SiO₂-haltigen Beschichtung auf einem Stent aus einem metallischen Grundkörper, umfassend die Schritte:
(i) Bereitstellen des Stents aus einem metallischen Grundkörper;
(ii) Kontaktieren einer Oberfläche des Grundkörpers mit einer wässrigen kolloiden Dispersion, die amorphe SiO₂-Partikel mit einer mittleren Teilchengröße im Bereich von 5 - 75 nm enthält; und
(iii) gleichzeitig oder im Anschluss an Schritt (ii), thermische Behandlung des Stents zumindest im Bereich der Kontaktfläche unter Ausbildung der SiO₂-haltigen Beschichtung.

11. Verfahren nach Anspruch 10, bei dem im Schritt (ii) eine wässrige kolloide Dispersion verwendet wird, die nur die amorphen SiO₂-Partikel enthält, und der Schritt (iii) so durchgeführt wird, dass sich ein Film aus SiO₂ bildet.

12. Verfahren nach Anspruch 10, bei dem im Schritt (ii) eine wässrige kolloide Dispersion verwendet wird, die Polyurethan und einen Polyisocyanat-Härter enthält, und der Schritt (iii) so durchgeführt wird, dass sich eine polymere Matrix aus Polyurethan bildet, in die Nanopartikel aus SiO₂ eingebettet sind.

13. Verfahren nach einem der Ansprüche 10 bis 12, bei dem der metallische Grundkörper des Stents eine poröse Oberfläche aufweist und Schritt (ii) so durchgeführt wird, dass die Dispersion in die Poren der porösen Oberfläche eindringt.
